# EUROPÄISCHE PATENTSCHRIFT

(11) **EP 1 150 720 B1**
(45) Veröffentlichungstag und Bekanntmachung des Hinweises auf die Patenterteilung: **14.01.2004**
(21) Anmeldenummer: 99957908.9
(22) Anmeldetag: 16.10.1999
(51) Int. Cl.: A61L 2/14

(54) **VERFAHREN UND VORRICHTUNG ZUR STERILISATION VON GEFÄSSEN ODER GEGENSTÄNDEN**
METHOD AND DEVICE FOR STERILIZING CONTAINERS OR OBJECTS
PROCEDE ET DISPOSITIF DE STERILISATION DE RECIPIENTS OU D'OBJETS

(30) Priorität: 01.02.1999 DE 19903935
(43) Veröffentlichungstag der Anmeldung: 07.11.2001
(73) Patentinhaber: ROBERT BOSCH GMBH, 70442 Stuttgart (DE)
(72) Erfinder: BURGER, Kurt, D-71292 Friolzheim (DE); WILKE, Bernd, D-71397 Leutenbach (DE); RAUSCHNABEL, Johannes, D-70197 Stuttgart (DE); HENKE, Sascha, D-71263 Weil Der Stadt (DE); VOIGT, Johannes, D-71229 Leonberg (DE)
(86) Internationale Anmeldenummer: PCT/DE1999/003324
(87) Internationale Veröffentlichungsnummer: WO 2000/045861

(56) Entgegenhaltungen:
- WO-A-98/51608
- DE-A- 3 129 997
- DE-A- 19 720 159
- GB-A- 1 098 693
- GB-A- 2 247 624

## Beschreibung

### Stand der Technik

Die Erfindung betrifft ein Verfahren zur Sterilisation vorzugsweise von Gefäßen und Vorrichtungen zur Durchführung des Verfahrens nach dem Oberbegriff des Hauptanspruchs.
Es ist hinlänglich bekannt, zur Beseitigung von schädlichen Mikroorganismen oder Keimen in Gefäßen in der Medizin oder der Lebensmitteltechnologie, z.B. bei Ampullen, Schnappdeckelgläser, Septengläser oder sog. Vials physikalische oder chemische Verfahren einzusetzen. Beispielsweise können bei einem Wasserdampfverfahren mit einer wässrigen Vorreinigung die Gefäße über einen vorgegebenen Zeitraum heißem Wasserdampf ausgesetzt werden. Die Dauer des Prozesses erfordert große Anlagen, um in den Fertigungsfluss integriert hohe Stückzahlen sterilisieren zu können. Die Sterilisation muß vollständig erfolgen, d.h. unter Abtötung sämtlicher Keime. Die sterilisierten Ampullen müssen dabei vor dem Befüllen außerdem getrocknet werden, wodurch mit den dafür benötigten Trocknereinheit das Anlagenvolumen zusätzlich vergrößert wird. Diese Wasserdampfsterilisation ist jedoch nicht in der Lage sog. pyrogene, d.h. entzündlich wirkende Abbauprodukte und Zellrestbestandteile von abgetöteten Keimen vollständig zu entfernen.

Aus der EP 0 377 799 A 1 ist ein Verfahren bekannt, bei dem zur Sterilisation von Gegenständen ein Plasma mit einer elektromagnetischen Strahlung mit einer Frequenz von etwa 2,45 GHz erzeugt wird. Der Gegenstand wird hierzu vollständig einem Niederdruckplasma ausgesetzt und in einer Erweiterung auch mit einer zusätzlichen Wärmequelle bestrahlt.

### Vorteile der Erfindung

Ein Verfahren zur Sterilisation von Gefäßen oder Gegenständen, mit der Anregung eines Plasmas in den Gefäßen oder an den Gegenständen durch elektromagnetische Schwingungen, wird gemäß der Erfindung mit den kennzeichnenden Merkmalen des Hauptanspruchs in vorteilhafter Weise derart ausgebildet, dass eine Plasmasterilisation derart durchgeführt wird, dass ein räumlich und zeitlich selektives Anregen des Plasmas durch eine getrennte Steuerung des Druckgradienten innerhalb und außerhalb des Gefäßes oder des Gegenstandes bewirkt wird, wodurch die Plasmasterilisation in verschiedenen Bereichen der Wände des Gefäßes oder der Gegenstände vorgenommen wird in denen durch einen ausreichenden Druck unterhalb des Atmosphärendrucks eine Plasmaanregung erfolgt.
Mit der Erfindung ist es auf einfachen Weise möglich die Keimabtötung und das vollständige Entfernen von Pyrogenen in einem Verfahren zu integrieren, wobei die Verfahrenssicherheit in hohem Maße gewährleistet ist. Die erfindungsgemäße Plasmasterilisation ermöglicht mit sehr kurzen Prozesszeiten bei der Durchführung des Verfahrens gleichzeitig die Abtötung von Keimen und die vollständige Beseitigung der eingangs erwähnten Pyrogene im Inneren wie auf der Außenwand der Gefäße. Eine abschließende Trocknung der Gefäße ist hierbei nicht notwendig.
Bei einer ersten Ausführungsform wird das Gefäß in eine Kammer geführt, in der zumindest nahezu ein Vakuum herstellbar ist. In das Innere des Gefäßes kann auf einfache Weise über eine von der Kammer abgeschirmte Zuleitung ein zur Anregung des Plasmas geeignetes Gas geführt werden, wobei der Gasdruckgradient im Inneren so eingestellt und gehalten wird, dass nur hier ein Plasma angeregt und eine vorgegebene Zeit aufrechterhalten wird. Der Gasdruckgradient und das Plasma im Inneren des Gefäßes werden bei dieser vorteilhaften Ausführungsform durch eine ausreichende Höhe des Druckwertes gegenüber dem Druckwert in der Kammer, auch mit einem vorgegebenen Abfluss des Abgases aus dem Gefäß in die Kammer, und einer anschließenden Absaugung aus der Kammer aufrecht erhalten.
Über die partiellen Druckwerte des Gases kann hierbei in vorteilhafter Weise die Anregung des Plasmas gesteuert werden. Bei einem zu geringem Druck können nicht genug Elementarteilchen angeregt bzw. ionisiert werden um eine Plasmaentladung aufrecht zu erhalten. Bei einem zu hohem Druck des Gases ist die mittlere freie Weglänge zu gering, um eine Aktivierung oder Anregung der Elementarteilchen zu bewirken.

Im einzelnen kann in einem ersten Verfahrensschritt die Kammer evakuiert werden und in einem zweiten Verfahrensschritt das Gas in das Gefäß zur Anregung des Plasmas im Inneren eingeleitet werden. In einem dritten Verfahrensschritt kann darüber hinaus anschließend das Gas in die Kammer geleitet werden zur Anregung des Plasmas in der Kammer und außen am Gefäß bei gleichzeitigem Erlöschen des Plasmas im inneren des Gefäßes.

Bei einer zweiten vorteilhaften Ausführungsform des erfindungsgemäßen Verfahrens wird das Gefäß in eine Kammer geführt, in die das zur Anregung des Plasmas geeignete Gas geführt wird. Im Inneren des Gefäßes ist nunmehr über eine von der Kammer abgeschirmte Zuleitung eine zumindest teilweise Evakuierung herstellbar, wobei der Gasdruckgradient im Inneren so eingestellt und gehalten wird, dass hier ein Plasma angeregt und eine vorgegebene Zeit aufrechterhalten wird.

Bei diesem gegenüber der ersten Ausführungsform des Verfahrens inversen Prozess wird somit der Gasdruckgradient und das Plasma im Inneren des Gefäßes durch eine ausreichende Tiefe des Druckwertes gegenüber dem Druckwert in der Kammer mit einem vorgegebenen Zufluss des Gases aus der Kammer in das Gefäß und einer anschließenden Absaugung aus dem Gefäß aufrecht erhalten.

Im einzelnen wird hier in einem ersten Verfahrensschritt die Kammer mit dem Gas versorgt und in einem zweiten Verfahrensschritt das Gefäß soweit evakuiert, dass über den Zufluss des Gases aus der Kammer die Anregung des Plasmas im Inneren erfolgt. In einem dritten Verfahrensschritt kann auch hier, wie in ähnlicher Weise oben erläutert, die Gaszufuhr in die Kammer gestoppt werden zur Anregung des Plasmas außen am Gefäß bei gleichzeitigen Erlöschen des Plasmas im Inneren des Gefäßes.
Dieses, zuletzt dargestellte, sog. inverse Prinzip hat den Vorteil, dass nur das kleinere Volumen, nämlich nur das Innere des zu sterilisierenden Gefäßes abgesaugt werden muss, während in der Kammer zunächst ein Grobvakuum ausreichend ist. Erst im zweiten Verfahrensschritt wird die Kammer abgesaugt.
Bei einer besonders vorteilhaften Vorrichtung zur Durchführung des erfindungsgemäßen Verfahrens sitzt das Gefäß in der Kammer auf einem als Gefäßhalterung dienenden, innen offenen Konus auf. Der Konus weist außen im Bereich des Sitzes des hier offenen Gefäßes eine Leckage-Nut auf und ist innen über eine Zuleitung mit einer außerhalb der Kammer liegenden Gaszufuhr oder Pumpe verbindbar. An die Kammer ist eine Pumpe zur Evakuierung und/oder eine Gaszufuhr für das anzuregende Gas anschließbar. Außen an der Kammer ist eine Plasmaquelle, vorzugsweise zur Abstrahlung von Mikrowellenenergie, angebracht. Die Frequenz der Mikrowellenstrahlung kann hierbei vorzugsweise auch in einem Bereich von 4,9 GHz liegen.
Um eine Vielzahl von Gefäßen zu sterilisieren können in vorteilhafter Weise die Gefäße in Glieder einer Kette zum Transport in die Kammer eingefädelt werden. Als Gefäßhalterung ist hier ein als Absaug- oder Gaszufuhrschiene dienendes Vierkant vorhanden, auf dem die Gefäße nahezu druckdicht mit einer vorgegebenen Leckage geführt werden. Das Vierkant ist dabei mit einer außerhalb der Kammer liegenden Gaszufuhr oder Pumpe, wie oben beschrieben, verbunden.
Bei einer weiteren automatisierbaren Vorrichtung sind vorteilhaft eine Vielzahl von Gefäßen in Löcher einer Transportbox zum Transport, gegebenenfalls mit einem Handhabungsautomaten oder Roboter, in die Kammer eingebracht. Die Gefäße sitzen in den Löchern mit ihren Öffnungen nahezu druckdicht mit einer vorgegebenen Leckage ein. Die Transportbox kann über einen Bodenflansch mit einer außerhalb der Kammer liegenden Gaszufuhr oder Pumpe wie oben verbunden werden.
Mit der Erfindung kann unter Einbeziehung der Vorrichtungen zur Durchführung des Sterilisationsverfahrens in vorteilhafter Weise das Anlagenvolumen bei hoher zu sterilisierender Stückzahl pro Zeiteinheit klein gehalten werden. Die Investitionskosten für die Vorrichtungen sind im Vergleich zu herkömmlichen Lösungen niedriger, wobei insbesondere ein kontinuierlicher Fertigungsfluss bei der Durchführung des Verfahrens realisierbar ist.
Es kann mit der Erfindung erreicht werden, dass eine nahezu sichere Kontrolle der Sterilisation auf einfache Weise durchführbar ist. Es braucht lediglich das Leuchten des Plasmas mit einfachen optischen Mitteln überwacht zu werden um eine definitive Aussage über die Sterilisation zu erhalten. Durch die Anwendung eines Niedertemperaturplasmas mit einem hierzu geeigneten Gas ist es auch möglich Kunststoffgefäße oder -gegenstände mit dem erfindungsgemäßen Verfahren zu sterilisieren, da die hier auftretenden Temperaturen in der Regel kleiner als 150 °C sind.
Diese und weitere Merkmale von bevorzugten Weiterbildungen der Erfindung gehen außer aus den Ansprüchen, einschließlich der rückbezogenen Unteransprüche, auch aus der Beschreibung und den Zeichnungen hervor, wobei die einzelnen Merkmale jeweils für sich allein oder zu mehreren in Form von Unterkombinationen bei der Ausführungsform der Erfindung und auf anderen Gebieten verwirklicht sein und vorteilhafte sowie für sich schutzfähige Ausführungen darstellen können, für die hier Schutz beansprucht wird.

### Zeichnung

Ausführungsbeispiele von Vorrichtungen zur Durchführung des erfindungsgemäßen Verfahrens zur Sterilisation von Gefäßen werden anhand der Zeichnung erläutert. Es zeigen:
Figur 1 eine schematische Ansicht einer Vorrichtung mit einem in einer Kammer angeordneten Gefäß, wobei in der Kammer ein Vakuum erzeugt wird;
Figur 2 eine schematische Ansicht einer Vorrichtung mit einem in einer Kammer angeordneten Gefäß, wobei im Gefäß ein Vakuum erzeugt wird;
Figur 3 eine schematische Ansicht eines Ausführungsbeispiels einer Transportvorrichtung für die zu sterilisierenden Gefäße und
Figur 4 eine schematische Ansicht einer Transportvorrichtung mit einer Transportbox für die zu sterilisierenden Gefäße.

### Beschreibung der Ausführungsbeispiele

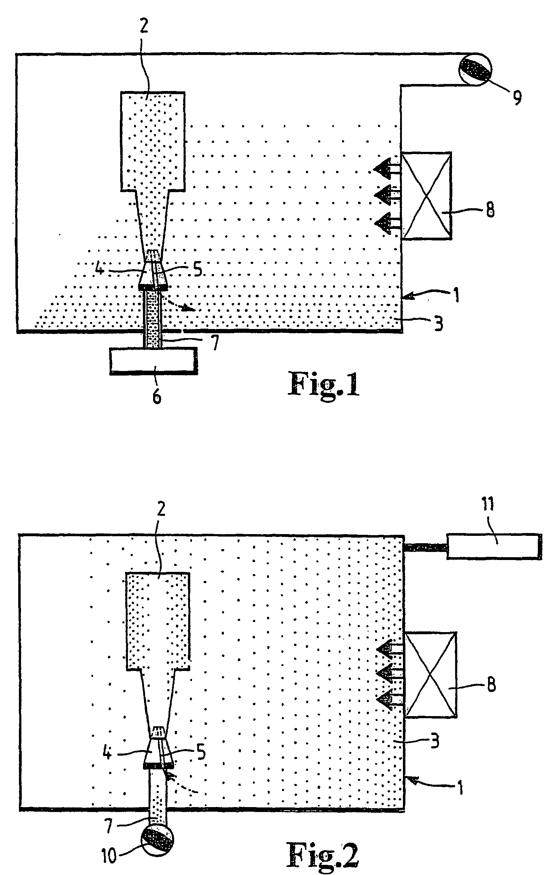

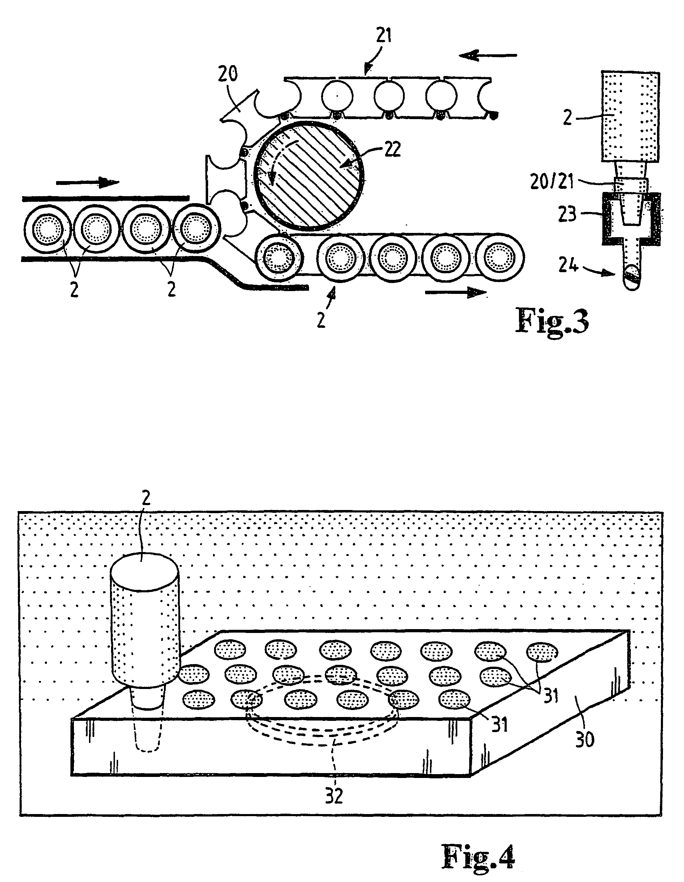

In Figur 1 ist ein erstes Ausführungsbeispiel einer Vorrichtung 1 zur Plasmasterilisierung von Gefäßen 2 dargestellt. In einer gegen die umgebende Atmosphäre abschließbaren Kammer 3 (hier Vakuumkammer) erfolgt der Sterilisationsprozess, wobei das Gefäß 2 auf einem Konus 4 als Gefäßhalterung gehalten wird. Der Konus 4 weist außen eine Leckage-Nut 5 auf, die somit zwischen dem inneren Rand der Öffnung des aufliegenden Gefäßes 2 und der Außenwand des Konusses 4 liegt und eine, wenn auch geringe, Gasströmung zwischen der Kammer 3 und dem Inneren des Gefäßes 2 ermöglicht. Durch den innen offenen Konus 4 ist über eine Zuleitung 7 von außerhalb der Kammer 3, gesteuert mit einem Durchflussregler 6, ein Gas in das Gefäß 2 zuführbar. Außen an der Kammer 3 ist stilisiert eine Plasmaquelle 8, z.B. zur Erzeugung elektromagnetischer Wechselfelder, gezeigt und es ist weiterhin eine Pumpe 9 zur Absaugung von Gas aus der Kammer 3 vorhanden.
Das Prinzip des hier angewendeten Sterilisationsverfahrens beruht auf einem an sich bekannten physikalischen Prozess, der zur Erzeugung eines Plasmas aus einem permanenten Gas führt, wobei die Atome des Gases durch eine geeignete Energiezufuhr in ein Gemisch von Elektronen und lonen umgewandelt werden. Die Energiezufuhr erfolgt hier bevorzugt durch die Beschleunigung der Ladungsträger der Elementarteilchen, insbesondere der Elektronen, in elektrischen Feldern, die dem Plasma von außen mit der Plasmaquelle 8 aufgeprägt werden.
Gemäß der erfindungsgemäßen Ausführungsbeispiele nach der Figur 1 und bei einem weiter unten beschriebenen zweiten Ausführungsbeispiel nach Figur 2 erfolgt dabei eine selektives Anregung des Plasmas. Durch zumindest zeitweise während. der Verfahrensschritte hervorgerufene unterschiedliche Gasdrücke im Inneren des Gefäßes 2 und in der umgebenden Kammer 3 kann das Plasma gezielt entweder innen oder außen angeregt werden. Bei einem zu geringem Druck können nicht genug Elementarteilchen angeregt bzw. ionisiert werden um eine Plasmaentladung aufrecht zu erhalten. Bei einem zu hohem Druck des Gases ist die mittlere freie Weglänge zu gering, um den Elementarteilchen zwischen zwei Stößen genug Beschleunigungsstrecke oder Beschleunigungszeit zur Aktivierung oder Anregung und damit zur Ionisation zu lassen.
Beim ersten Ausführungsbeispiel nach der Figur 1 wird die Kammer 3 mit der Pumpe 9 so abgepumpt, daß der Gasdruck in ihrem Inneren der Kammer 3 für die Anregung eines Plasmas zu gering ist. In der Kammer 3 sind die zu sterilisierenden Gefäße 2 so gehalten, dass sie direkt mit ihrer Öffnung formschlüssig auf dem Konus 4 sitzen. Über die Zuleitung 7 strömt von außerhalb der Kammer 3, gesteuert durch den Durchflussregler 6, ein definierter Gasstrom aus beispielsweise Sauerstoff, gefilterte Luft, Wasserdampf, Wasserstoffperoxid-Dampf, Argon, Stickstoff Tetrafluormethan, Schwefelhexafluorid o.ä., durch das Innere des Konusses 4 in das Gefäß 2. Dieser Gasstrom ist so eingestellt, dass im Inneren des Gefäßes 2 der Druck so groß wird, dass ein Plasma angeregt werden kann.
Um einen gewünschten Druckgradienten bzw. bei einem bestimmten Druck einen definierten Gasaustausch im Gefäß 2 nach der Figur 1 zu erhalten, wird über die Leckage-Nut 5 außen am Konus 4 das Gas, bzw. das Abgas des Plasmas, aus dem Gefäß 2 in die Kammer 3 abgesaugt. In Abhängigkeit von der durch den Konus 4 zuströmenden Gasmenge und dem Leitwert der Leckage-Nut 5 ist dabei der Druck des Gases im Gefäß 2 einstellbar. Möglich ist hier auch eine gesteuerte Leckage-Nut, beispielsweise mit einem Ventil. Das in die Kammer 3 durch die Leckage-Nut 5 zugeströmte Abgas wird dann mit der Pumpe 9 zur Erhaltung der Druckverhältnisse aus der Kammer 3 abgesaugt, wodurch somit ein Plasma selektiv nur im Inneren des zu sterilisierenden Gefäßes 2 erzeugt ist.
In einer besonders vorteilhaften weitergebildeten Ausführung dieses selektiven Sterilisationsprozesses wird die Kammer 3 nach der Figur 1 zuerst vollständig abgepumpt ohne einen Gasfluss in das Gefäß 2 oder die Kammer 3 einströmen zu lassen. Anschließend wird der Durchflussregler 6 zur Erzeugung eines definierten Gasstroms geöffnet, der in das zu sterilisierenden Gefäß 2 einfließt und im Inneren des Gefäßes 2 ein Plasma zündet, vorteilhafterweise durch Mikrowelleneinstrahlung der Plasmaquelle 8.
Wenn die gewünschte Sterilisationswirkung im Inneren des Gefäßes 2 erreicht ist, kann optional noch in der Kammer 3, also außerhalb des zu sterilisierenden Gefäßes 2, der Gasdruck erhöht werden, indem ein definierter Gasstrom desselben Gases, bzw. Gasgemisches, das durch die Zuleitung 7 fließt, oder gegebenenfalls auch durch einen zusätzlichen, hier nicht dargestellten Einlass in die Kammer 3 eingelassen wird, so dass ein Plasma auch hier angeregt werden kann. Das Plasma erlischt dabei im Inneren des Gefäßes 2 und wird außerhalb in der Kammer 3 angeregt. Mit diesem Verfahrensschritt lässt sich somit das Gefäß 2 auch an der, der Kammer 3 zugewandten Außenwand sterilisieren.
Dieser zuvor beschriebene Vorgang des nach außen Springens der Plasmabildung entsteht dadurch, dass ein Plasma sich nach außen abschirmt, d.h. die eingestrahlte Energie soweit absorbiert, dass die Energie außerhalb dieses Plasmas nicht ausreicht, um ein weiteres Plasma anzuregen. So schirmt das äußere Plasma in der Kammer 3 sich gegenüber der abgetrennten Gasatmosphäre im Inneren des zu sterilisierenden Gefäßes 2 ab und verhindert dort die Anregung eines Plasmas. Ist der Druck dabei in der Kammer 3 zu gering, aber im Inneren des Gefäßes 2 für ein Plasma ausreichend, so wird die eingestrahlte Energie im inneren Plasma des Gefäßes 2 weitgehend absorbiert. Da die Energie der Plasmaquelle 8 aber zuerst in die Kammer 3 eingestrahlt wird und erst dann durch die Wand des zu sterilisierenden Gefäßes 2, unter Umständen gedämpft, in das Innere des Gefäßes 2 gelangt, wird im anderen Fall das Plasma sofort außen am Gefäß 2 entstehen, wenn in der Kammer 3 der Gasdruck dafür ausreichend ist.
Bei dem zweiten Ausführungsbeispiel nach Figur 2 wird das anhand der Figur 1 beschriebene Prinzip umgekehrt; die im wesentlichen gleichwirkenden Bauteile sind jedoch mit den gleichen Bezugszeichen wie bei der Figur 1 versehen. Das zu sterilisierende Gefäß 2 wird hier direkt über den Konus 4 mit einer Pumpe 10 zur Bildung eines Vakuums im Gefäß 2 abgesaugt. Die Durchführung des Sterilisationsverfahrens mit diesem Ausführungsbeispiel erfolgt nun in der Weise, dass nachdem die Kammer 3 mit dem Sterilisationsgas, bzw. -Gasgemisch durch eine Gaszufuhr 11 geflutet ist, das zu sterilisierenden Gefäß 2 soweit abgepumpt wird, dass ein Plasma durch Einstrahlung eines elektromagnetischen Feldes, vorteilhafterweise eines Mikrowellenfeldes, im Inneren des Gefäßes 2 angeregt ist, während der Druck außerhalb des zu sterilisierenden Gefäßes 2 in der Kammer 3 für ein Plasma zu hoch ist (vgl. die Erläuterung weiter oben). Das Gas strömt dabei durch die Leckage-Nut 5 am Konus 4 der Gefäßhalterung in das Gefäß 2 ein. Das Abgas im Gefäß 2 kann nunmehr durch den Konus 4 und die Zuleitung 7 mit der Pumpe 10 abgesaugt werden.
In einem anschließenden Verfahrensschritt wird die Kammer 3 zur Sterilisation der. Außenflächen des Gefäßes 2 soweit abgesaugt, daß das Plasma außen in der Kammer 3 brennt. Dies kann dadurch erfolgen, daß die Frischgaszufuhr mit dem Durchflussregler der Gaszufuhr 11 gestoppt wird und das Gas aus der Kammer 3 über die Leckage-Nut 5 am Konus 4 nach und nach abgesaugt wird bis der entsprechende Druck in der Kammer 3 erreicht ist. Alternativ ist dies auch mit hier nicht dargestellten Pumpen zu bewerkstelligen, mit denen die Kammer 3 zusätzlich evakuiert werden kann.
Anhand der zuvor beschriebenen Ausführungsbeispiele ist eine Sterilisation von einzelnen Gefäßen in einer relativ einfachen Vorrichtung erläutert worden. Das erfindungsgemäße Verfahren ist jedoch auch in einen, zumindest teilweise automatisierten Ablauf integrierbar, bei dem eine Vielzahl von Gefäßen 2 in Reihe kontinuierlich oder in Batches sequentiell, aber in einem von der jeweiligen Fertigungslinie vorgegebenen Takt, in die Kammer 3 ein- und ausgeschleust werden.
Im Falle von Ausführungsbeispielen zur Durchführung eines solchen Verfahrens nach Figuren 3 und 4 werden einige Einschleustechniken für die Gefäße 2 beschrieben, die den großen Drucksprung zwischen der umgebenden Atmosphäre und dem Vakuum in der Kammer 3 für ein relativ großes Volumen ermöglichen. Dies kann generell entweder eine mechanische Schleuseneinrichtung wie Türe oder Schieber oder eine differentiell gepumpte Anlagenkonfiguration sein, bei der durch eine permanent vorhandene Öffnung die Gefäße 2 eingeschleust werden, wobei die Öffnung, die, um eine minimale Leckage zu erreichen, möglichst dem Umriss der einzuschleusenden Gefäße 2 entsprechen sollte. Die Pumpenkonfiguration muß dabei extrem stark ausgeführt werden, um im Inneren der Vakuumanlage im hier größeren Volumen der Kammer 3 das notwendige Vakuum zu erreichen, wobei bei mit einer Kombination mit der Verfahrensvariante nach der Figur 2 eine wesentlich geringere Pumpleistung ausreicht, da nur das kleine Volumen der Gefäße 2 absaugt werden muß.
Beim Ausführungsbeispiel nach der Figur 3 lassen sich die zu sterilisierenden Gefäße 2 aus der Linie Stück für Stück in Glieder 20 einer Kette 21 einfädeln, die umlaufend um eine Umlenkrolle 22 den Transport in die Kammer 3 (vgl. die Figuren 1 und 2) und aus dieser hinaus übernimmt. Darüber hinaus sind die Kettenglieder 20 so ausgeformt, daß sie die Abdichtung der Gefäße 2 gegen die Druckstufe Grobvakuum/Feinvakuum übernehmen.
Zur Verdeutlichung ist im rechten Teil der Figur 3 ein Schnitt durch ein Gefäß 2 gezeigt, das in der Kette 21 bzw. in einem Kettenglied 20 liegt. Die Kette 21 wird in einem Längsschlitz, dessen Schlitzbreite dem Durchmesser der zu sterilisierenden Gefäße 2 angepasst ist, eines Vierkantprofils 23 geführt, das den einzelnen Gefäßen 2 als gemeinsame Absaugleitung dient und ebenfalls die Abdichtung gegen die Druckstufe übernimmt. Da hier beide Abdichtungselemente, nämlich die Kette 21 und das Vierkantprofil 23, Leckagen aufweisen, muss eine ausreichende Pumpleistung einer Pumpe 24 zur Verfügung gestellt werden. Die eigentliche Vorrichtung kann bei einem Grobvakuum oder sogar atmosphärischem Druck betrieben werden.
In dem zweiten Ausführungsbeispiel zur Sterilisation einer Vielzahl von Gefäßen 2 nach der Figur 4 erfolgt die Plasmasterilisation der Gefäße 2 in Gruppen, die in eine Transportbox 30 eingefügt sind. Die einzelnen Gefäße 2 können beispielsweise von einem Handhabungsautomaten in die Transportbox 30 gesteckt werden, die z.B. ein rechteckiger Behälter aus Edelstahl sein kann, in dessen Deckblech Löcher 31 eingestanzt sind. Der Durchmesser dieser Löcher 31 ist dabei so gewählt, daß die konischen Gefäße 2 (z.B. Glas- oder Kunststoffampullen), die in die Löcher 31 gesteckt werden, diese quasi abdichten.
Das Bodenblech des Edetstahlbehälters der Transportbox 30 besitzt hier einen Flansch 32, über den die Ankopplung an die Gaszuführung entsprechend der Ausführung der Figur 1 oder an den Pumpenflansch nach der Ausführung der Figur 2 erfolgt. Die so bestückte Transportbox 30 wird nun über eine hier nicht dargestellte Tür oder ein Plattenventil in eine Vorkammer der Vakuumanlage, die auch die Kammer 3 beinhaltet, eingeschleust, die Türe geschlossen und die Vorkammer abgepumpt. Anschließend erfolgt in der Vakuumanlage der Transfer aus der Vorkammer in die Kammer 3 oder einen entsprechenden Kammerbereich, in dem die Absaugung erfolgt. Die Absaugung wird solange durchgeführt bis ein Druckbereich erreicht wird, in dem die Zündung des Plasmas erfolgen kann. Die Ausschleusung der Gefäße 2 erfolgt hierbei in einer entsprechenden Art und Weise wie das Einschleusen.

## Patentansprüche

1. Verfahren zur Sterilisation von Gefäßen oder Gegenständen, mit
- der Anregung eines Plasmas in den Gefäßen oder an den Gegenständen durch elektromagnetische Schwingungen, **dadurch gekennzeichnet, dass**
- eine Plasmasterilisation derart durchgeführt wird, dass ein räumlich und zeitlich selektives Anregen des Plasmas durch eine getrennte Steuerung des Druckgradienten innerhalb und außerhalb des Gefäßes (2) oder des Gegenstandes bewirkt wird, wodurch die Plasmasterilisation in verschiedenen Bereichen der Wände des Gefäßes (2) oder der Gegenstände vorgenommen wird in denen durch einen ausreichenden Druck unterhalb des Atmosphärendrucks eine Plasmaanregung erfolgt.

2. Verfahren nach Anspruch 1, **dadurch gekennzeichnet, dass**
- das Gefäß (2) in eine Kammer (3) geführt wird, in der zumindest nahezu ein Vakuum herstellbar ist und dass
- in das Innere des Gefäßes (2) über eine von der Kammer (3) abgeschirmte Zuleitung (7) ein zur Anregung eines Plasmas geeignetes Gas geführt wird, wobei ein Gasdruckgradient im Inneren so eingestellt und gehalten wird, dass hier ein Plasma angeregt und eine vorgegebene Zeit aufrechterhalten wird.

3. Verfahren nach Anspruch 2, **dadurch gekennzeichnet, dass**
- der Gasdruckgradient und das Plasma im Inneren des Gefäßes (2) durch eine ausreichende Höhe des Druckwertes gegenüber dem Druckwert in der Kammer (3) auch mit einem vorgegebenen Abfluss des Abgases aus dem Gefäß (2) in die Kammer (3) und einer anschließenden Absaugung aus der Kammer (3) aufrecht erhalten wird.

4. Verfahren nach Anspruch 2 oder 3, **dadurch gekennzeichnet, dass**
- in einem ersten Verfahrensschritt die Kammer (3) evakuiert wird und
- in einem zweiten Verfahrensschritt das Gas in das Gefäß (2) zur Anregung des Plasmas im Inneren eingeleitet wird.

5. Verfahren nach Anspruch 4, **dadurch gekennzeichnet, dass**
- in einem dritten Verfahrensschritt ein Gas zur Anregung eines Plasmas in die Kammer (3) geleitet wird zur Anregung eines Plasmas in der Kammer (3) und damit außen am Gefäß (2) bei gleichzeitigen Erlöschen des Plasmas im Inneren des Gefäßes (2).

6. Verfahren nach Anspruch 1, **dadurch gekennzeichnet, dass**
- das Gefäß (2) in eine Kammer (3) geführt wird, in die ein zur Anregung eines Plasmas geeignetes Gas geführt wird, und dass
- im Inneren des Gefäßes (2) über eine von der Kammer (3) abgeschirmte Zuleitung (7) eine zumindest teilweise Evakuierung herstellbar ist wobei ein Gasdruckgradient im Inneren so eingestellt und gehalten wird, dass hier ein Plasma angeregt und eine vorgegebene Zeit aufrechterhalten wird.

7. Verfahren nach Anspruch 6, **dadurch gekennzeichnet, dass**
- der Gasdruckgradient und das Plasma im Inneren des Gefäßes (2) durch eine ausreichende Tiefe des Druckwertes gegenüber dem Druckwert in der Kammer (3) auch mit einem vorgegebenen Zufluss des Gases aus der Kammer (3) in das Gefäß (2) und einer anschließenden Absaugung aus dem Gefäß (2)aufrecht erhalten wird.

8. Verfahren nach Anspruch 6 oder 7, **dadurch gekennzeichnet, dass**
- in einem ersten Verfahrensschritt die Kammer (3) mit dem Gas versorgt wird und dass
- in einem zweiten Verfahrensschritt das Gefäß (2) soweit evakuiert wird, dass über den Zufluss des Gases aus der Kammer (3) die Anregung des Plasmas im Inneren erfolgt.

9. Verfahren nach Anspruch 8, **dadurch gekennzeichnet, dass**
- in einem dritten Verfahrensschritt die Gaszufuhr in die Kammer (3) gestoppt wird zur Anregung eines Plasmas in der Kammer (3) und damit außen am Gefäß (2) bei gleichzeitigen Erlöschen des Plasmas im Inneren des Gefäßes (2).

10. Vorrichtung zur Durchführung des Verfahren nach einem der vorhergehenden Ansprüche, **dadurch gekennzeichnet, dass**
- das Gefäß (2) in der Kammer (3) auf einem als Gefäßhalterung dienenden, innen offenen Konus (4) aufsitzt, wobei der Konus (4) außen im Bereich des weitgehend dichtenden Sitzes des Gefäßes (2) eine Leckage-Nut (5) aufweist und innen über eine Zuleitung (7) mit einer außerhalb der Kammer (3) liegenden Gaszufuhr (6) oder Pumpe (10) verbindbar ist, dass
- an die Kammer (3) eine Pumpe (9) und/oder eine Gaszufuhr (11) anschließbar ist und dass
- außen an der Kammer (3)eine Plasmaquelle (8) angebracht ist.

11. Vorrichtung nach Anspruch 10, **dadurch gekennzeichnet, dass**
- die Leckage-Nut (5) hinsichtlich des Gasdurchflusses steuerbar ist.

12. Vorrichtung zur Durchführung des Verfahren nach einem der Ansprüche 1 bis 9, **dadurch gekennzeichnet, dass**
- eine Vielzahl von Gefäßen (2) in Glieder (20) einer Kette (21) zum Transport in die Kammer (3) einbringbar sind, wobei als Gefäßhalterung ein als Absaug- oder Gaszufuhrschiene dienendes Vierkant (23) angeordnet ist, auf dem die Gefäße (2) nahezu druckdicht mit einer vorgegebenen Leckage geführt werden und das Vierkant (23) mit einer außerhalb der Kammer (3) liegenden Gaszufuhr (6) oder Pumpe (10) verbindbar ist, dass
- an die Kammer (3) eine Pumpe (9) und/oder eine Gaszufuhr (11) anschließbar ist und dass
- außen an der Kammer (3) eine Plasmaquelle (8) angebracht ist.

13. Vorrichtung zur Durchführung des Verfahren nach einem der Ansprüche 1 bis 9, **dadurch gekennzeichnet, dass**
- eine Vielzahl von Gefäßen (2) in Löcher (31) einer Transportbox (30) zum Transport in die Kammer (3) einbringbar sind, wobei die Gefäße (2) mit ihren Öffnungen nahezu druckdicht mit einer vorgegebenen Leckage einsitzen und die Transportbox (30) über einen Bodenflansch (32) mit einer außerhalb der Kammer (3) liegenden Gaszufuhr (6) oder Pumpe (10) verbindbar ist, dass
- an die Kammer (3) eine Pumpe (9) und/oder eine Gaszufuhr (11) anschließbar ist und dass
- außen an der Kammer (3)eine Plasmaquelle (8) angebracht ist.

14. Vorrichtung nach einem der Ansprüche 10 bis 13, **dadurch gekennzeichnet, dass**
- die zu sterilisierenden Gefäße (2) oder die Gegenstände aus Glas oder Kunststoff sind.

## Claims

1. Method for sterilizing containers or objects, comprising
- excitation of a plasma in the containers or at the objects by means of electromagnetic oscillations, **characterized in that**
- the plasma sterilization is carried out in such a manner that spatially and temporally selective excitation of the plasma is effected by separate control of the pressure gradient inside and outside the container (2) or the object, with the result that the plasma sterilization is performed in various regions of the walls of the container (2) or of the objects in which plasma excitation takes place as a result of a pressure sufficiently below atmospheric pressure.

2. Method according to Claim 1, **characterized in that**
- the container (2) is guided into a chamber (3) in which at least a virtual vacuum can be produced, and **in that**
- a gas which is suitable for exciting a plasma is passed into the interior of the container (2) via a feedline (7) which is shielded from the chamber (3), a gas pressure gradient being set and maintained in the interior such that a plasma is excited and maintained for a predetermined time in this interior.

3. Method according to Claim 2, **characterized in that**
- the gas pressure gradient and the plasma in the interior of the vessel (2) are maintained by a sufficient level of the pressure compared to the pressure in the chamber (3) even when there is a predetermined flow of the off-gas out of the vessel (2) into the chamber (3) followed by extraction by suction from the chamber (3).

4. Method according to Claim 2 or 3, **characterized in that**
- in a first method step, the chamber (3) is evacuated, and
- in a second method step the gas is introduced into the container (2) in order to excite the plasma in the interior.

5. Method according to Claim 4, **characterized in that**
- in a third method step, a gas for exciting a plasma is passed into the chamber (3) in order to excite a plasma in the chamber (3) and therefore on the outside of the container (2) while at the same time extinguishing the plasma in the interior of the container (2).

6. Method according to Claim 1, **characterized in that**
- the container (2) is passed into a chamber (3) into which a gas which is suitable for exciting a plasma is passed, and **in that**
- at least partial evacuation can be produced in the interior of the container (2) by means of a feedline (7) which is shielded from the chamber (3), a gas pressure gradient being set and maintained in the interior in such a way that a plasma is excited and maintained for a predetermined time in this interior.

7. Method according to Claim 6, **characterized in that**
- the gas pressure gradient and the plasma in the interior of the container (2) are maintained by making the pressure sufficiently low compared to the pressure in the chamber (3) even with a predetermined flow of the gas out of the chamber (3) into the container (2) followed by extraction of this gas from the container (2) by suction.

8. Method according to Claim 6 or 7, **characterized in that**
- in a first method step, the chamber (3) is supplied with the gas, and **in that**
- in a second method step, the container (2) is evacuated until the plasma is excited in the interior as a result of the incoming flow of the gas from the chamber (3).

9. Method according to Claim 8, **characterized in that**
- in a third method step, the supply of gas into the chamber (3) is stopped in order to excite a plasma in the chamber (3) and therefore on the outside of the container (2) while at the same time extinguishing the plasma in the interior of the container (2).

10. Device for carrying out the method according to one of the preceding claims, **characterized in that**
- the container (2) in the chamber (3) is seated on a cone (4) which serves as a container holder and is open on the inside, the cone (4), on the outer side, having a leakage groove (5) in the region of the substantially sealing seat of the container (2) and on the inner side being capable of being connected via a feedline (7) to a pump (10) or gas feed (6) located outside the chamber (3), **in that**
- a pump (9) and/or a gas feed (11) can be connected to the chamber (3), and **in that**
- a plasma source (8) is arranged on the outside of the chamber (3).

11. Device according to Claim 10, **characterized in that**
- the leakage groove (5) is controllable with regard to the through-flow of gas.

12. Device for carrying out the method according to one of Claims 1 to 9, **characterized in that**
- a multiplicity of containers (2) can be introduced into links (20) of a chain (21) to be transported into the chamber (3), a square (23), which is used as a suction or gas feed rail, being arranged as a container holder, on which square the containers (2) are guided in a virtually pressure-tight manner with a predetermined leakage, and the square (23) can be connected to a pump (10) or gas feed (6) located outside the chamber (3), **in that**
- a pump (9) and/or gas feed (11) can be connected to the chamber (3), and **in that**
- a plasma source (8) is arranged on the outside of the chamber (3).

13. Device for carrying out the method according to one of Claims 1 to 9, **characterized in that**
- a multiplicity of containers (2) can be introduced into holes (31) in a conveyor box (30) to be transported into the chamber (3), the containers (2) with their openings being seated in a virtually pressure-tight manner with a predetermined leakage, and it being possible for the conveyor box (30) to be connected via a base flange (32) to a pump (10) or gas feed (6) located outside the chamber (3), **in that**
- a pump (9) and/or a gas feed (11) can be connected to the chamber (3), and **in that**
- a plasma source (8) is arranged on the outside of the chamber (3).

14. Device according to one of Claims 10 to 13, **characterized in that**
- the containers (2) which are to be sterilized or the objects are made from glass or plastic.

## Revendications

1. Procédé de stérilisation de récipients ou d'objets avec
- excitation d'un plasma dans les récipients ou contre les objets par oscillations électromagnétiques,
**caractérisé en ce que**
- une stérilisation au plasma est exécutée de manière telle qu'une excitation sélective du plasma, sur un plan spatial et temporel, est réalisée par une commande séparée du gradient de pression à l'intérieur et à l'extérieur du récipient (2) ou de l'objet, ce par quoi la stérilisation au plasma est entreprise en différentes zones des parois du récipient (2) ou de l'objet, dans lesquelles par une pression suffisante en dessous de la pression atmosphérique, une excitation du plasma s'effectue.

2. Procédé selon la revendication 1,
**caractérisé en ce que**
- le récipient (2) est conduit dans une chambre (3) dans laquelle au moins un quasi vide peut être créé et
- à l'intérieur du récipient (2) un gaz adapté à l'excitation du plasma est conduit par une canalisation (7) d'amené blindée par rapport à la chambre (3), un gradient de pression à l'intérieur étant établi et maintenu de manière telle qu'ici un plasma est excité et conservé pendant une durée prédéterminée.

3. Procédé selon la revendication 2,
**caractérisé en ce que**
- le gradient de pression de gaz et le plasma à l'intérieur du récipient (2) sont conservés par une hauteur suffisante de la valeur de pression par rapport à la valeur de pression dans la chambre (3) également avec un écoulement prédéterminé du gaz d'échappement hors du récipient (2) dans la chambre (3) et une aspiration successive hors de la chambre (3).

4. Procédé selon la revendication 2 ou 3,
**caractérisé en ce que**
- dans une première étape de procédé, un vide est créé dans la chambre (3) et
- dans une deuxième étape de procédé, le gaz dans le récipient (2) pour l'excitation du plasma est introduit à l'intérieur.

5. Procédé selon la revendication 4,
**caractérisé en ce que**
- dans une troisième étape de procédé, un gaz pour l'excitation d'un plasma est conduit dans la chambre (3) pour l'excitation d'un plasma dans la chambre (3) et, de ce fait, à l'extérieur, contre le récipient (2), en éteignant simultanément le plasma à l'intérieur du récipient (2).

6. Procédé selon la revendication 1,
**caractérisé en ce que**
- le récipient (2) est conduit dans une chambre (3) dans laquelle est conduit un gaz adapté à l'excitation d'un plasma et
- à l'intérieur du récipient (2), par une canalisation (7) d'amené blindée par rapport à la chambre (3), un vide au moins partiel peut être créé, un gradient de pression de gaz étant établi et maintenu à l'intérieur de manière telle qu'ici un plasma est excité et conservé pendant une durée prédéterminée.

7. Procédé selon la revendication 6,
**caractérisé en ce que**
- le gradient de pression de gaz et le plasma à l'intérieur du récipient (2) sont conservés par une profondeur suffisante de la valeur de pression par rapport à la valeur de pression dans la chambre (3) également avec un apport prédéterminé du gaz hors de la chambre
(3) dans le récipient (2) et une aspiration successive hors du récipient (2).

8. Procédé selon la revendication 6 ou 7,
**caractérisé en ce que**
- dans une première étape de procédé, la chambre (3) est alimentée avec ce gaz et
- dans une deuxième étape de procédé, un vide est créé dans le récipient (2) jusqu'à ce que, par l'apport du gaz de la chambre (3), l'excitation du plasma à l'intérieur se réalise.

9. Procédé selon la revendication 8,
**caractérisé en ce que**
- dans une troisième étape de procédé, l'apport de gaz dans la chambre (3) est arrêté pour l'excitation d'un plasma dans la chambre (3) et, de ce fait, à l'extérieur, contre le récipient (2), en éteignant simultanément le plasma à l'intérieur du récipient (2).

10. Dispositif pour l'exécution du procédé selon l'une quelconque des revendications précédentes,
**caractérisé en ce que**
- le récipient (2) dans la chambre (3) est placé sur un cône (4) ouvert à l'intérieur, servant de support de récipient, le cône (4) comportant une rainure (5) de fuite à l'extérieur, dans la zone de l'assise largement étanche du récipient (2), et pouvant être relié à l'intérieur, par une canalisation (7) d'amené, avec une arrivée (6) de gaz reposant à l'extérieur de la chambre (3) ou une pompe (10),
- une pompe (9) et/ou une amenée (11) de gaz peut/peuvent être raccordée(s) à la chambre (3), et
- à l'extérieur, à la chambre (3), une source (8) de plasma est montée.

11. Dispositif selon la revendication 10,
**caractérisé en ce que**
- la rainure (5) de fuite est commandable sur le plan du débit gazeux.

12. Dispositif pour l'exécution du procédé selon l'une quelconque des revendications 1 à 9,
**caractérisé en ce que**
- une pluralité de récipients (2) dans les maillons (20) d'une chaîne (21) peut, pour le transport, être introduite dans la chambre (3), avec comme support de récipient, un carré (23) servant de rail d'aspiration ou d'apport de gaz, sur lequel les récipients (2) sont guidés, quasiment étanches à la pression, avec une fuite prédéterminée, et le carré (23) peut être relié à une amenée (6) de gaz ou une pompe (10) reposant à l'extérieur de la chambre (3),
- une pompe (9) et/ou une amenée (11) de gaz peut/peuvent être raccordée(s) à la chambre (3), et
- à l'extérieur, à la chambre (3), une source (8) de plasma est montée.

13. Dispositif pour l'exécution du procédé selon l'une quelconque des revendications 1 à 9,
**caractérisé en ce que**
- une pluralité de récipients (2) peut se loger dans des trous (31) d'un casier (30) de transport pour le transport dans la chambre (3), les récipients (2) se positionnant par leur ouverture de manière quasiment étanche à la pression avec une fuite prédéterminée, et le casier (30) de transport étant relié par une bride de fond (32) avec une amenée (6) de gaz ou une pompe (10) reposant à l'extérieur de la chambre (3),
- une pompe (9) et/ou une amenée (11) de gaz peut/peuvent être raccordée(s) à la chambre (3), et
- à l'extérieur, à la chambre (3), une source (8) de plasma est montée.

14. Dispositif selon l'une quelconque des revendications 10 à 13,
**caractérisé en ce que**
- les récipients (2) à stériliser ou les objets sont en verre ou en matière synthétique.
